# EUROPEAN PATENT SPECIFICATION

(11) **EP 2 790 650 B1**
(45) Date of publication and mention of the grant of the patent: **24.05.2017**
(21) Application number: 11811220.0
(22) Date of filing: 15.12.2011
(51) Int. Cl.: A61K 8/34, A61K 8/49, A61Q 5/02, A61Q 11/00, A61Q 19/10, A61Q 17/00, C11D 9/26, C11D 7/26, C11D 3/48

(54) **SOLUBILIZED MAGNOLOL ANALOGS**
AUFGELÖSTE MAGNOLOL-ANALOGA
ANALOGUES DU MAGNOLOL SOLUBILISÉS

(43) Date of publication of application: 22.10.2014
(73) Proprietor: Colgate-Palmolive Company, New York, NY 10022 (US)
(72) Inventor: HOURIGAN, Regina, Metuchen, New Jersey 08840 (US); MASTRULL, Jeffrey, Flemington, New Jersey 08822 (US); MATTAI, Jairajh, Piscataway, New Jersey 08854 (US); MASTERS, James, Ringoes, New Jersey 08551 (US)
(74) Representative: Wichmann, Hendrik
(86) International application number: PCT/US2011/065016
(87) International publication number: WO 2013/089716

(56) References cited:
- WO-A1-2010/042313
- WO-A1-2011/106492
- WO-A1-2011/131439
- WO-A2-2011/055139
- WO-A2-2011/106493
- FR-A1- 2 953 406
- US-A1- 2003 194 385
- US-A1- 2006 140 880
- "Solubilising the most challenging actives with... Super Refined Arlasolve (TM) DMI", , 1 January 2007 (2007-01-01), XP055194194, Retrieved from the Internet: URL:http://www.crodalubricants.com/downloa d.aspx?s=133&m=doc&id=240 [retrieved on 2015-06-08]
- 'Isosorbide dimethyl ether', [Online] Retrieved from the Internet: <URL:http://www.chemicalbook.com/ProductChe micalPropertiesCB0457121_EN.htm> [retrieved on 2015-07-02]

## Description

### FIELD OF THE INVENTION

Disclosed are solubilized magnolol analogs.

### BACKGROUND OF THE INVENTION

Magnolol analogs, such a propyl magnolol, isopropyl magnolol, butyl magnolol, and isobutyl magnolol, are known to have anti-bacterial activities and they are also shown to be capable of reducing the expression of pro-inflammatory mediators in oral tissues. The problem with using these magnolol analogs is their solubility in typical personal care, oral care, or home care compositions. Their use has been limited by their solubility. It would be desirable to solubilize these analogs to increase their use in personal, oral, or home care compositions. The problem is finding materials that can solubilize these analogs. Even in a given class of material, not all members of the class are effective at solubilizing these analogs.

WO2011/106492 and WO2011/106493 describe oral care compositions comprising one or more active ingredients found in magnolia extract, or a synthetic analogue thereof.

### BRIEF SUMMARY OF THE INVENTION

The present invention provides a composition according to claim 1. Optional features are set out in the dependent claims. The present disclosure provides a composition comprising a solubilized magnolol analog comprising at least one magnolol analog chosen from n-propyl magnolol, isopropyl magnolol, and isobutyl magnolol, and dimethyl isosorbide.

Further areas of applicability of the present invention will become apparent from the detailed description provided hereinafter.

### DETAILED DESCRIPTION OF THE INVENTION

The following description of the preferred embodiment(s) is merely exemplary in nature and is in no way intended to limit the invention, its application, or uses.

Disclosed is a composition comprising a solubilized magnolol analog comprising at least one magnolol analog chosen from propyl magnolol, isopropyl magnolol, and isobutyl magnolol, and dimethyl isosorbide.

Propyl magnolol is 5,5'-di-n-propylbiphenyl-2,2'-diol, butyl magnolol is 5,5'-di-n-butylbiphenyl-2,2'-diol, isopropyl magnolol is 5,5'-di-isopropylbiphenyl-2,2'-diol, and isobutyl magnolol is 5,5'-di-isobutylbiphenyl-2,2'-diol.

The dimethyl isosorbide is capable of solubilizing up to 200 g per liter of neat propyl magnolol or isopropyl magnolol, up to 100 g per liter of isobutyl magnolol, or up to 50 g per liter of butyl magnolol.

Dimethyl isosorbide is available as Arlasove™ DMI from Croda.

It was surprising that dimethyl isosorbide was able to solubilize these analogs. Many other solubilizers, such as PEG-7 glyceryl cocoate, poloxamer 124, PPG-2 hydroxyethyl cocoamide, PPG-5 laureth-5 (Eumulgin™ ES), PEG-8/SMDI copolymer, isopropyl myristate, or C12-15 alkyl benzoate are not able to solubilize isobutyl magnolol.

The amount of magnolol analog in the composition can be any desired amount. In certain embodiments, the amount is 0.01 to 5% by weight of the composition. In other embodiments, the amount is at least 0.05, at least 0.1, at least 0.2, at least 0.3, at least 0.4, at least 0.5, or at least 1% by weight up to 5% by weight of the composition. In other embodiments, the amount is any of the foregoing minimum amounts up to 4, up to 3, up to 2, or up to 1% by weight of the composition. The weight of the dimethyl isosorbide is then the amount to solubilize the analog with the minimum amount of the dimethyl isosorbide being based on the maximum solubility of the analog in the dimethyl isosorbide. In certain embodiments, the amount of the magnolol analog is 0.1, 0.2, 0.3, 0.4, or 0.5% by weight.

These solubilized analogs are useful in personal care, oral care, and home care compositions. Examples of personal care compositions include, but are not limited to, body wash/shower gel, liquid hand cleanser, bar soap, shampoo, conditioner, antiperspirant/deodorants, and cosmetics. Examples of oral care compositions include, but are not limited to, dentifrices, toothpastes, tooth powders, prophylaxis pastes, mouth rinses, lozenges, gums, gels, paints, confectionaries, and denture cleaners. Examples of oral care compositions that can include solubilized magnolol analogs can be found in WO2011/106492.
Examples of home care compositions include, but are not limited to, dish liquids, dish pastes, hard surface cleaners, fabric conditioners, and laundry detergents.

In certain embodiments, the magnolol analog can be present in a body wash/shower gel, liquid hand cleanser, or shampoo in which each of these compositions include a surfactant. The magnolol analog can also be included in a soap (fatty acid soap), which can be in the shape of a bar soap.

### Examples

The following are non-limiting prophetic examples of compositions that can include solubilized magnolol analogs.

**Liquid Cleanser (Body Wash or Liquid Hand Soap)**

| **Ingredient Name** | **% Wt. Range** | **% Wt. Range** | **% Wt. Range** |
|---|---|---|---|
| Propyl magnolol or isopropyl magnolol | 0.01-1% | 0 | 0 |
| Butyl magnolol | 0 | 0.01-1% | 0 |
| Isobutyl magnolol | 0 | 0 | 0.01-1% |
| Dimethyl isosorbide | At least 5 times the weight of the magnolol analog | At least 10 times the weight of the magnolol analog | At least 20 times the weight of the magnolol analog |
| Polyquaternium-7 | 0-0.25 | 0-0.25 | 0-0.25 |
| SO₃Na Pareth 145-2EO Sulfate | 8-12 | 8-12 | 8-12 |
| Cocamidopropyl Betaine | 2.5-7 | 2.5-7 | 2.5-7 |
| Decyl Glucoside | 0-2 | 0-2 | 0-2 |
| Demineralized Water and minors | Q.S. | Q.S. | Q.S. |
| Total Materials | 100 | 100 | 100 |

**Bar Soap**

| **Ingredient Name** | **% Wt. Range** | **% Wt. Range** | **% Wt. Range** |
|---|---|---|---|
| Propyl magnolol or isopropyl magnolol | 0.01-1% | 0 | 0 |
| Butyl magnolol | 0 | 0.01-1% | 0 |
| Isobutyl magnolol | 0 | 0 | 0.01-1% |
| Dimethyl isosorbide | At least 5 times the weight of the magnolol analog | At least 10 times the weight of the magnolol analog | At least 20 times the weight of the magnolol analog |
| Fatty acid soap | 75-85 | 75-85 | 75-85 |
| Demineralized Water and minors | Q.S. | Q.S. | Q.S. |
| Total Materials | 100 | 100 | 100 |

**Oral Care Composition**

| **Ingredient** | **Weight %** | **Weight %** | **Weight %** |
|---|---|---|---|
| Purified water | Q.S. | Q.S. | Q.S. |
| Sorbitol | 19.45 | 19.45 | 19.45 |
| Glycerin | 20 | 20 | 20 |
| Sodium CMC-12 type USP | 1.1 | 1.1 | 1.1 |
| Iota carrageenan (LB 9505) | 0.4 | 0.4 | 0.4 |
| Sodium saccharin-USP | 0.3 | 0.3 | 0.3 |
| Sodium fluoride | 0.24 | 0.24 | 0.24 |
| Zeodent-115-dental type silica abrasive | 8.5 | 8.5 | 8.5 |
| Zeodent-165-synthetic amorphous PPT silica | 3 | 3 | 3 |
| Dental type silica sylodent XWA650 | 10 | 10 | 10 |
| Titannium dioxide (TiO2) | 0.5 | 0.5 | 0.5 |
| Sodium lauryl sulphate powder-NF | 1.5 | 1.5 | 1.5 |
| Flavor | 1 | 1 | 1 |
| Propyl magnolol or isopropyl magnolol | 0.01-1% | 0 | 0 |
| Butyl magnolol | 0 | 0.01-1% | 0 |
| Isobutyl magnolol | 0 | 0 | 0.01-1% |
| Dimethyl isosorbide | At least 5 times the weight of the magnolol analog | At least 10 times the weight of the magnolol analog | At least 20 times the weight of the magnolol analog |
| Total | 100 | 100 | 100 |

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

## Claims

1. A composition comprising a solubilized magnolol analog comprising:
at least one magnolol analog chosen from n-propyl magnolol, isopropyl magnolol, and isobutyl magnolol; and
dimethyl isosorbide.

2. The composition of claim 1, wherein the at least one magnolol analog is present in an amount of 0.01 to 5% by weight of the composition.

3. The composition of claim 1 or claim 2 further comprising a surfactant.

4. The composition of any preceding claim further comprising soap.

5. The composition of claim 4 in the form of a bar soap.

6. The composition of claim 1 in the form of a liquid hand cleanser.

7. The composition of any of claims 1 to 3 in the form of an oral care composition.

## Patentansprüche

1. Zusammensetzung, umfassend ein solubilisiertes Magnolol-Analogon, umfassend:
mindestens ein Magnolol-Analogon, ausgewählt aus n-Propyl-Magnolol, Isopropyl-Magnolol und Isobutyl-Magnolol; und
Dimethylisosorbid.

2. Die Zusammensetzung nach Anspruch 1, wobei das mindestens eine Magnolol-Analogon in einer Menge von 0,01 bis 5%, bezogen auf das Gewicht der Zusammensetzung, vorliegt.

3. Die Zusammensetzung nach Anspruch 1 oder Anspruch 2, weiterhin umfassend ein oberflächenaktives Mittel.

4. Die Zusammensetzung nach einem beliebigen vorangegangenen Anspruch, weiterhin umfassend Seife.

5. Die Zusammensetzung nach Anspruch 4 in der Form eines Seifenstücks.

6. Die Zusammensetzung nach Anspruch 1 in der Form eines flüssigen Handreinigers.

7. Die Zusammensetzung nach einem beliebigen der Ansprüche 1 bis 3 in der Form einer Mundpflege-Zusammensetzung.

## Revendications

1. Composition comprenant un analogue de magnolol solubilisé comprenant :
au moins un analogue de magnolol choisi parmi le n-propyl magnolol, l'isopropyl magnolol et l'isobutyl magnolol ; et
du diméthyl isosorbide.

2. Composition selon la revendication 1, dans laquelle le ou les analogues de magnolol sont présents en une quantité de 0,01 à 5 % en poids de la composition.

3. Composition selon la revendication 1 ou revendication 2, comprenant en outre un tensioactif.

4. Composition selon l'une quelconque des revendications précédentes, comprenant en outre un savon.

5. Composition selon la revendication 4, sous la forme d'un pain de savon.

6. Composition selon la revendication 1 sous la forme d'un nettoyant liquide pour les mains.

7. Composition selon l'une quelconque des revendications 1 à 3 sous la forme d'une composition de soin buccal.
